# EUROPEAN PATENT SPECIFICATION

(11) **EP 3 363 365 B1**
(45) Date of publication and mention of the grant of the patent: **09.02.2022**
(21) Application number: 18156865.0
(22) Date of filing: 10.12.2012
(51) Int. Cl.: A61B 8/00, A61B 8/08, A61B 8/12

(54) **AUTOMATIC IMAGING PLANE SELECTION FOR ECHOCARDIOGRAPHY**
AUTOMATISCHE AUSWAHL EINER BILDGEBUNGSEBENE FÜR DIE ECHOKARDIOGRAFIE
SÉLECTION DE PLAN D'IMAGERIE AUTOMATIQUE POUR ÉCHOCARDIOGRAPHIE

(30) Priority: 12.12.2011 US 201161569450 P
(43) Date of publication of application: 22.08.2018
(62) Divisional of application: 12818929.7
(73) Proprietor: Koninklijke Philips N.V., 5656 AG Eindhoven (NL); Philips GmbH, 22335 Hamburg (DE)
(72) Inventor: RADULESCU, Emil George, 5656 AE Eindhoven (NL); WEESE, Juergen, 5656 AE Eindhoven (NL); SALGO, Ivan, 5656 AE Eindhoven (NL)
(74) Representative: Philips Intellectual Property & Standards

(56) References cited:
- US-A1- 2005 096 538
- US-A1- 2008 009 722
- US-A1- 2010 260 398
- BAHBIBI RAHMATULLAH ET AL: "Automated Selection of Standardized Planes from Ultrasound Volume", 18 September 2011 (2011-09-18), MACHINE LEARNING IN MEDICAL IMAGING, SPRINGER BERLIN HEIDELBERG, BERLIN, HEIDELBERG, PAGE(S) 35 - 42, XP019166526, ISBN: 978-3-642-24318-9 * page 36, paragraph 3 * * page 39, paragraph 1 * * figure 1 *
- ORDERUD ET AL: "Automatic alignment of standard views in 3D echocardiograms using real-time tracking", SPIE, PO BOX 10 BELLINGHAM WA 98227-0010 USA, vol. 7265, 7 February 2009 (2009-02-07), XP040495310,

## Description

### FIELD OF THE INVENTION

The present invention relates to imaging anatomy and, more particularly, to, based on the anatomy in the imaging, modifying the imaging.

### BACKGROUND OF THE INVENTION

Accurate anatomical measurements are needed pre-treatment and for diagnosis of a number of cardiac conditions. The gold standard treatment for aortic stenosis is surgical replacement of the aortic valve. Open heart surgery is required, but is not an option for some elderly patients. A recently-developed enormously less invasive alternative is Transcatheter Aortic Valve Implantation (TAVI). Via a catheter, a replacement valve is advanced intravenously and disposed at the location of the current, faulty valve. In preparation, the diameter of the aortic valve annulus is determined. As another example, the diameter of the ascending aorta is calculated to assess the potential for an aneurysm.

Medical imaging is a non-invasive method for making the anatomical measurements. In medical image processing applications, various processing tasks are typically performed on the images. One specific processing task, which is a fundamental task in many image processing applications, is the segmentation of a specific organ. For many organs, segmentation can successfully be performed with shape-constrained deformable models. They are based on a mesh structure with a topology which remains unchanged during adaptation to the image being segmented. Model-based segmentation has been considered very efficient for a wide variety of simple to complex organs (e.g., bones, liver, and heart with nested structures). Indeed, recent results show that this technique enables fully automatic segmentation of complex anatomical structures such as the heart.

Commonly-assigned U.S. Patent Publication Number 2008/0304744 to Peters et al., (hereinafter "the '744 application'"), adapts an anatomical model to a three-dimensional (3D) ultrasound image, and encodes the adapted model for automatic subsequent execution of specific image-processing tasks, such as localization and tracking of target anatomical structures.

US 2005/0096538 A1 discloses a medical imaging system which automatically acquires two-dimensional images representing a user-defined region of interest despite motion. The plane of acquisition is updated or altered adaptively as a function of detected motion. The user-designated region of interest is then continually scanned due to the alteration in scan plane position. A multi-dimensional array is used to stabilize imaging of a region of interest in a three-dimensional volume. The user defines a region of interest for two-dimensional imaging.

US 2010/0260398 A1 discloses a systems and methods which provide volume imaging by implementing survey and target imaging modes. According to embodiments, a survey imaging mode is implemented to provide a volume image of a relatively large survey area. A target of interest is preferably identified within the survey area for use in a target imaging mode. Embodiments implement a target imaging mode to provide a volume image of a relatively small target area corresponding to the identified target of interest. The target imaging mode preferably adapts the beamforming, volume field of view, and/or other signal and image processing algorithms to the target area.

US 2008/0009722 A1 discloses to generate a plurality of images corresponding to a plurality of different planes in a volume during scanning or in real-time with acquisition of ultrasound data. The volume scan data is searched by a processor to identify desired views. Multiple stand or predetermined views are generated based on plane positioning within the volume by the processor.

### SUMMARY OF THE INVENTION

The non-ionizing, high resolution and high tissue contrast advantages of echocardiography enable fast diagnosis and reliable guidance for interventional applications.

Fast diagnosis requires expedited acquisition of standard views. This is difficult as the user needs to orient the ultrasound transducer probe to optimally capture the standard views (e.g. 2D images, X-planes which are two views that cross the apex (axially closest point) of the image, or selected volumetric acquisition).

In addition, the trade-off between beam density, volume size and frame rate in 3D echocardiography makes accurate quantification challenging when looking at complex structures such as the aortic root or aortic aneurysms.

Accurate measurements require high beam density which is hard to attain if imaging a large volume at high frame rate. Usually the mitigation is to collect only 2D images or a set of 2D images (e.g. X-planes) with a correct orientation and perform measurements on those images. The orientation of the planes has to be then carefully selected.

In the case of an aneurysm of the ascending aorta, for instance, selection of a correct imaging plane is important. This selection is, however, highly dependent on the orientation of the probe, the anatomy of the ascending aorta and the skill of the operator. For example, the position of a transesophageal (TEE) ultrasound probe relative to specific targeted cardiac anatomy varies from patient to patient.

Cardiac image segmentation of the whole heart in 3D ultrasound adversely impacts beam density and/or frame rate, as noted herein above. Specifically, scanning with a two-dimensional TEE or TTE transducer array in the azimuthal and elevation directions is performed at a rate that is limited by the need to receive the return echo of the beam before issuing the next, adjacent beam. Ultrasound is slow in comparison to other imaging modalities, traveling through body tissue at merely 1540 meters per second. Therefore, at a typical display refresh rate of about 25 Hz, beam density, i.e., the number of beams through a sector, is relatively low. For example, at 20 to 30 Hz only a few hundred transmit beams may be available. Spatial resolution is consequently impacted. this patent application, "real time" means without intentional delay, given the processing limitations of the system and the time required to accurately process the data.

The invention is defined by the independent claims. Advantageous embodiments are provided in the dependent claims.

### BRIEF DESCRIPTION OF THE DRAWINGS

FIGs. 1A and 1B are conceptual diagrams of volume imaging and plane imaging, respectively;
FIGs. 2A and 2B are conceptual diagrams of volume imaging and portion imaging, respectively;
FIG. 3 is a conceptual diagram of movement-based interruption of portion imaging and responsive re-execution of volume imaging;
FIG 4 is an illustration of display views and measurement-initializing indicia; and
FIG. 5 is an operational flow chart for an imaging-volume-portion selection device.

### DETAILED DESCRIPTION OF EMBODIMENTS

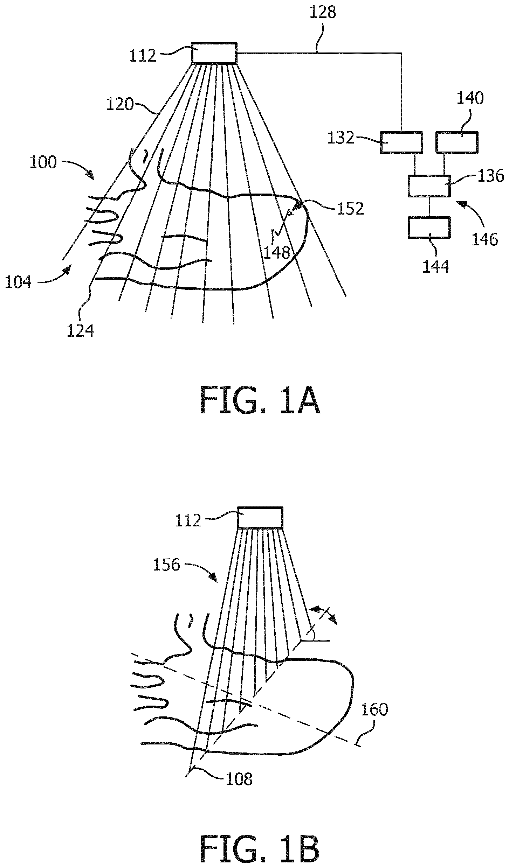

FIGs. 1A and 1B show, by way of illustrative and non-limitative example, live imaging of a volume 100 such as an entire heart 104 of a human or animal, that imaging being subsequently reduced to live imaging within a single imaging plane 108. The imaging, in both cases, is performed by a probe 112. For cardiac applications, the probe 112 can be a TEE probe, for intracorporeal use, or a TTE probe. The TEE probe is advanced down the esophagus into position for imaging. In the TTE case, an imaging end of the probe 112 will typically be handheld and controlled by a sonographer, cardiologist or radiologist. In the TEE case, the probe 112 will typically be controllable by one or more pull cables for steering and a multiplane probe will be rotatable within the esophagus manually or by a motor. This maneuvering can be done under image guidance afforded by an imaging window in the probe 112 through which ultrasound imaging is performed. Once the probe 112 is properly positioned, volume imaging can proceed. The TEE or TTE probe 112 will have a two-dimensional transducer array. For simplicity of illustration, scanning along a single dimension is shown in FIG. 1A. Thus, a return echo from a first beam 120 is awaited during the pendency of an acquisition time gate, and then a second beam 124 in the scan is issued. Since in the TEE case the probe 112 is internally disposed closer to the imaging volume 100, the time of flight of the ultrasound is reduced. Accordingly, the beam density, and thus spatial resolution, is higher with the TEE probe 112 than with a transthoracic (TTE) probe used externally, and typically manually. In addition, increased imaging clarity can also be attributed to the smaller attenuation, i.e., over a smaller distance. Yet, the principles discussed herein apply also to a TTE probe. Likewise, body tissue other than the heart, such as a fetus is within the intended scope of what is proposed herein.

The probe 112 is connected, by a cable 128 to an image acquisition module 132. The latter is communicatively connected to a processor 136 having a computer readable medium. The processor 136 is also communicatively connected to a display device 140 and a user interface unit 144. These are all components of an imaging-volume-portion selection device 146 in the current example.

As in the '744 application, a cardiac mesh model is adapted to the acquired 3D volume image. The adaptation may be carried out for a particular phase of the beating heart. Alternatively, it may be carried out separately for multiple phases and may be repeated, in this sense, periodically and continually. At this point, and/or later on, anatomical landmark information previously encoded on the model can be associated to respective locations in the 3D image. As an additional application, a target blood vessel 148 of the mesh, if located in the imaging, allows the determined vessel orientation with respect to the probe 112 to be used in automatic calculation of the Doppler angle 152.

In real time, one or more portions of the volume 100 are now automatically selected for subsequent live imaging, at greater beam density 156. For example, the imaging plane 108, or X-plane, through the volume 100 may comprise a selected volume portion. If more than one portion is selected, these may be contained within respective imaging planes 108, 160.

The selection involves adjusting, for the live portion imaging, the beamforming parameters just-previously derived for the volume imaging. The adjustment is based on the anatomy in the acquired volume imaging to improve imaging in one or more targeted views, such as standard diagnostic views or views facilitating accurate caliper measurements between anatomical landmarks or points.

Selection is based on an application-dependent optimal-view criterion. For general diagnostic imaging, the selection is performed so as to achieve standard views with respect to the anatomy. Examples of standard views of the heart are the four-chambers (or "apical") view, the parasternal long-axis view, the parasternal short-axis view, and the subcostal view. Additional examples include arbitrary views related to anatomy imaged or multimodality aligned views. For aortic aneurysm measurement, the aorta is identified by applying the model. The imaging plane 108 is selected perpendicular to the centerline of the aorta and corresponding to the maximum diameter, when shifting the viewing plane along the centerline. As a second example, TAVI planning involves using the model to identify the aortic valve. Information encoded in the model, as in the '744 application, such as a ring around the aortic valve annulus is associated with the 3D image. Then, a pair of imaging planes 108, 160 is selected to optimally cut the ring such that the resulting 2D images allow for proper aortic valve annulus diameter measurements, the plane selection inherently involving choosing an orientation. Along with display of the 2D image, indicia overlaid on the image show the clinician where to make the measurement. The automatic selection alleviates the above-noted typical problem of manual selection being highly dependent on the orientation of the probe, the patient-specific anatomy, and the skill of the operator. The clinician may alternatively define and store imaging planes via the user interface unit 144. This may be done interactively with display of the imaged volume on the display device 140. The selected planes 108, 160 are displayed as live imaging acquired with optimal beam density 156 and consequent improved imaging, and with measurement-initializing indicia overlaid.

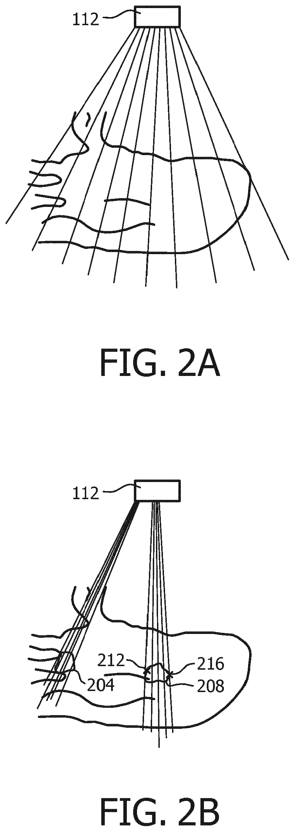

In FIGs. 2A and 2B, selected portions 204, 208 of the volume 100 which extend beyond an imaging plane are imaged live in 3D. As demonstrated in the exemplary embodiment in FIGs. 2A and 2B, the portion imaging, of which plane imaging is a special case, is likewise at higher beam density. Illustratively, indicia 212, 216, which are here anatomical points, are associated with the selected portion 208. The indicia 212, 216 may be displayed to initialize measurement there between by the clinician viewing the display device 140.

Over time, relative movement may occur with respect to respective positions of an imaging probe 112 and body tissue being imaged. Also, in the case of TTE, the patient, who may be asked to hold his or her breath, or the clinician may inadvertently move.

The relative movement can cause the live imaging to move out of alignment, i.e., out of conformance with the beamforming parameters previously calculated responsive to the portion selection.

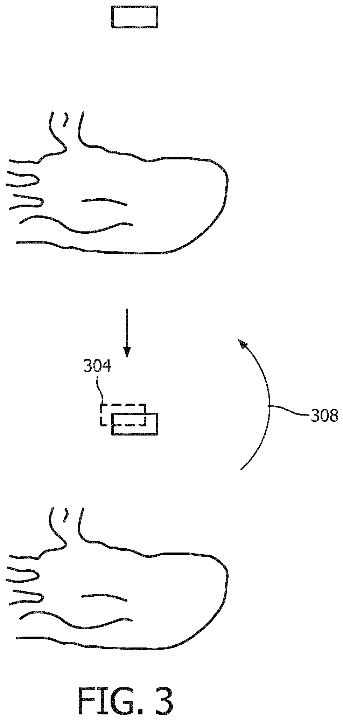

As represented by the broken line, FIG. 3 shows movement 304 of the imaging probe 112 relative to the volume 100 at a given phase of the beating heart. This is detectable by comparing, for a given phase, a current image to previous, stored images. The comparison is made during portion imaging, of which plane imaging is a special case. The comparisons can be made periodically, or continuously, to detect movement. If movement is detected, portion imaging is interrupted 308 and the volume acquisition is re-executed. Thus, anatomy recognition that adapts the model to the 3D imaging leads, based on the re-executed volume acquisition, to selecting of one or more portions, and imaging of the selected portion(s). Re-execution of volume, and then portion, imaging may alternatively be designed to occur periodically, irrespective of any relative movement, but as a precaution in case of movement.

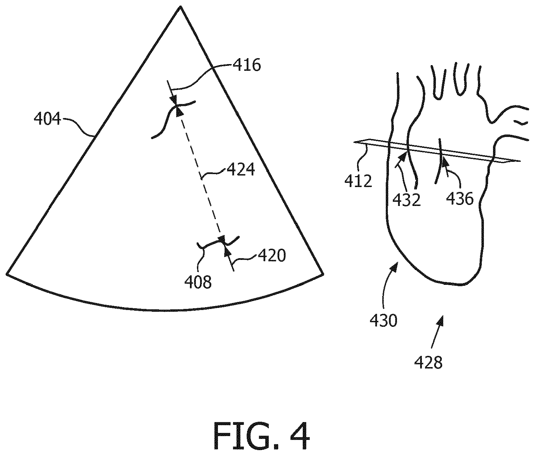

FIG. 4 shows one example of a two-dimensional live image 404 of an ascending aorta 408, the image existing in the imaging plane 412 selected. Due to potential aneurysm, an accurate measurement is needed. Overlaid on the image 404, are measurement-initializing indicia 416, 420 in the form of arrows. A caliper measurement of the diameter 424 of the ascending aorta, at its widest, is shown. Locations of the indicia 416, 420 are derived after model adaption identifies the ascending aorta. Information encoded at a location, on the mesh, that corresponds to the aorta leads to a search for the maximum diameter along the centerline of the aorta.

A perspective image 428 appears on a display screen alongside the two-dimensional image 404. The "cutting" or imaging plane 412 is visible as is the adjacent body tissue 430 of the volume 100. Indicia 432, 436 correspond to the indicia 416, 420 in the two-dimensional image 404.

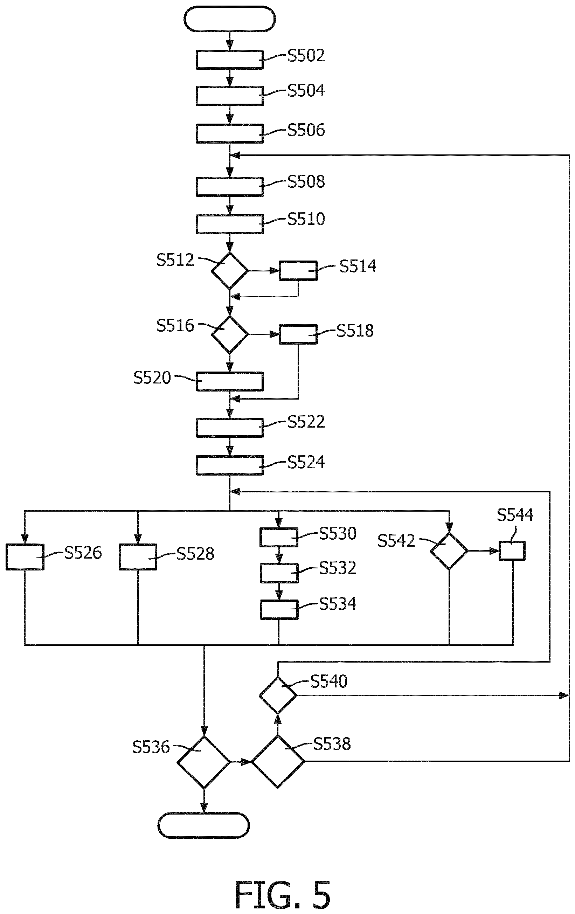

In preparation for operating the imaging-volume-portion selection device 146, and as indicated in the exemplary flow chart of FIG. 5, two steps (steps S502, S504) can be carried out in either order or concurrently. A clinical application, such as selecting standard views, arbitrary views related to anatomy imaged, multimodality aligned views, aortic valve measurement or ascending aorta measurement, is selected (step S502). An anatomical model, such as a cardiac mesh model, is encoded with information for measurement initialization (step S504). A 3D TEE/TTE probe 112 is then maneuvered into position for the volume imaging (step S506). This can be aided by 2D or 3D imaging feedback.

At this point the procedure, automatically and without the need for user intervention, is ready to commence. Alternatively, at this point, the operator can actuate a control to initiate further processing. This may be in reaction to what the operator sees on the screen in the 3D display. Volumetric data of the volume 100 is acquired during live imaging (step S508). The anatomical model is fitted to data acquired in the volume imaging, i.e., to the acquired image (step S510). The fitting may occur after every one or two heart beats progressively, for example, or may be delayed until a full acquisition that results in a view composed of several heart beats. If a Doppler parameter is to be calculated (step S512), it is calculated (step S514). In any event, if one or more portions 204, 208 are to be selected by the operator (step S516), the portions, such as imaging planes, are, by means of the user interface unit 144, defined and stored (step S518). The stored portions are used in the same way the automatically selected portions are used and once, stored, can be re-selected by navigating to the predefined choice. In the real time path, the one or more portions 204, 208 are automatically derived based on the adapted mesh and the information encoded thereon (step S520). Once the selection, automatic or manual, is complete, beamforming parameters and other image settings for the portion imaging are computed (step S522). The encoded information is associated to the respective one or more imaging locations (step S524). At this point, the one or more selected portions 108, 160, 204, 208 are collectively imaged live with high beam density and frame rate, affording more accurate measurements than 3D volume imaging would allow (step S526).

Display of the imaging may commence in real time (step S528). The model is applied to the data acquired in the current portion imaging (step S530). The encoded information, and indicia, is associated to image locations (step S532). The indicia are displayed (step S534). At this stage, the operator can, by a user control, adjust the image, e.g., the plane tilt, in a return to step S516.

If the portion imaging is still ongoing (step S536), but motion of the anatomy relative to the probe 112 is detected (step S538), processing returns to step S508 to re-acquire volumetric data of the volume 100. If the portion imaging is still ongoing (step S540), no motion is detected (step S538), and the re-execution of volume imaging acquisition is periodic (step S540), processing will likewise return to step S508 if the current period has expired and to just after step S524 otherwise so as to continue live portion imaging.

The display of images in steps S528 and S534 can be frozen, automatically or by the operator, for caliper measurement. Images can also be made part of a cineloop. If the portions are planes (step S542), the perspective view 428 of the anatomy cut by the plane is shown alongside the live or frozen portion imaging display (step S544).

The Doppler parameter computation of step S514 can alternatively be performed based on the portion imaging.

As an alternative to or in addition to image display that commences in real time, the volume imaging and portion imaging, cineloops derived therefrom, and the anatomical model mesh can be stored in Digital Imaging and Communication in Medicine (DICOM) format for subsequent analysis and quantification.

Based on anatomy recognition from three-dimensional ultrasound live imaging of a volume, one or more portions of the volume are selected in real time. In further real time response, live imaging or the portion(s) is performed with a beam density and overall image quality higher than that used in the volume imaging. The one or more portion may be one or more imaging plane selected for optimal orientation in making an anatomical measurement or optimal orientation for standard views for diagnostic imaging. Arbitrary views related to anatomy imaged or multimodality aligned views may as well be considered. The recognition can be based on an anatomical model, such as a cardiac mesh model. The model may be pre-encoded with information that can be associated with image locations to provide the basis for portion selection, and for placement of indicia displayable for initiating measurement within an image provided by the live portion imaging. A single TEE or TTE imaging probe may be used throughout. On request, periodically or based on detected motion of the probe with respect to the anatomy, the whole process can be re-executed, starting back from volume acquisition.

Applications of the automatic imaging volume portion selection technology include cardiac imaging with a 3D TTE/TEE probe. Examples are imaging of the aorta valve and ascending aorta and, specifically, aortic root measurements in preparation for aortic valve replacement and accurate measurements of the ascending aorta. An additional example is the optimal planes selection for standard views required in diagnostic imaging. Arbitrary views related to anatomy imaged or multimodality aligned views are as well additional examples.

While the invention has been illustrated and described in detail in the drawings and foregoing description, such illustration and description are to be considered illustrative or exemplary and not restrictive; the invention is not limited to the disclosed embodiments.

For example, distances between the aortic valve plane and coronary ostia can be calculated based on the techniques disclosed hereinabove. As another example, interruption of the portion imaging to re-execute volume imaging acquisition may be performed on request by the operator.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Any reference signs in the claims should not be construed as limiting the scope.

A computer program can be stored momentarily, temporarily or for a longer period of time on a suitable computer-readable medium, such as an optical storage medium or a solid-state medium. Such a medium is non-transitory only in the sense of not being a transitory, propagating signal, but includes other forms of computer-readable media such as register memory, processor cache, RAM and other volatile memory.

A single processor or other unit may fulfill the functions of several items recited in the claims. The mere fact that certain measures are recited in mutually different dependent claims does not indicate that a combination of these measures cannot be used to advantage.

## Claims

1. An image processing device (146) configured for:
three-dimensional live imaging of a volume with sequentially acquiring multiple beams such that said three-dimensional live imaging of the volume is performed with a spatial density (156) of said beams;
based on anatomy recognition (S510) from said three-dimensional live imaging of the volume (100), automatically and without need for user intervention, selecting, in response to said three-dimensional live imaging of the volume, one or more portions of said volume; and
automatically and without need for user intervention, performing, in response to the selection and with a beam density higher than said spatial density, live imaging of the one or more selected portions;
wherein said device is further configured for automatically and without need for user intervention, selectively interrupting (308) the live imaging of the one or more selected portions to re-execute the three-dimensional live imaging of the volume (S508) and, based on the re-executed three-dimensional live imaging of the volume, said recognition, said selecting and said live imaging of the one or more selected portions.

2. The device of claim 1, one or more imaging planes (108, 160) respectively comprising said one or more portions.

3. The device of claim 1, configured such that said interrupting occurs periodically (S540).

4. The device of claim 1, configured for detecting relative movement with respect to respective positions of an imaging probe and body tissue (S538), said interrupting being triggered based on the detected movement.

5. The device of claim 1, the volume imaging comprising ultrasound imaging (120) and/or cardiac imaging (104).

6. The device of claim 1, said selecting being based on an optimal-view criterion.

7. The device of claim 6, said criterion being based on making a targeted anatomical measurement from an image to be produced by the live imaging of the one or more selected portions (S526).

8. The device of claim 6, said selecting choosing, according to said criterion, an optimal orientation.

9. The device of claim 1, further configured for, automatically and without need for user intervention, calculating a Doppler angle (152) based on applying an anatomical model to at least one of the live imaging of the one or more selected portions and the three-dimensional live imaging of the volume.

10. The device of claim 1, said selecting being such as to optimize a measurement of distance (424), between predefined anatomical points, within body tissue represented by data acquired in the three-dimensional live imaging of the volume.

11. The device of claim 1, configured for, based on anatomy recognition, deriving a measurement-initializing indicium (416, 420, 432, 436), and for displaying the derived indicium to initialize image-based measurement within an image produced by the live imaging of the one or more selected portions.

12. The device of claim 11, said deriving comprising applying an anatomical model to at least one of said one or more selected portions (204, 208).

13. The device of claim 1, configured for said selecting such that a portion from among said one or more portions spans a predefined anatomical landmark (424) within body tissue (408) that is represented by data acquired in the three-dimensional live imaging of the volume.

14. The device of claim 1, said selecting choosing an viewing plane perpendicular to a centerline of an aorta and corresponding to a maximum diameter, when shifting the viewing plane along the centerline.

15. A computer readable medium (136) for a device for three-dimensional live imaging of a volume with sequentially acquiring multiple beams such that said three-dimensional live imaging of the volume is performed with a spatial density (156) of said beams, said medium comprising instructions executable by a processor for carrying out a plurality of acts, among said plurality there being the acts of:
based on a result of anatomy recognition from said three-dimensional live imaging of a volume, selecting, automatically and without need for user intervention, in response to said three-dimensional live imaging of the volume, one or more portions of said volume; and
automatically and without need for user intervention, performing, in response to the selection and with a beam density higher than said spatial density, live imaging of the one or more selected portions;
wherein among said plurality of acts there is the further act of automatically and without need for user intervention, selectively interrupting (308) the live imaging of the one or more selected portions to re-execute the three-dimensional live imaging of the volume (S508) and, based on the re-executed three-dimensional live imaging of the volume, said recognition, said selecting and said live imaging of the one or more selected portions.

## Patentansprüche

1. Bildverarbeitungsvorrichtung (146), die konfiguriert ist, zum:
dreidimensionale Live-Bildgebung eines Volumens mit sequentieller Erfassung mehrerer Strahlen, so dass die dreidimensionale Live-Bildgebung des Volumens mit einer räumlichen Dichte (156) der Strahlen durchgeführt wird;
basierend auf Anatomieerkennung (S510) aus der dreidimensionalen Live-Bildgebung des Volumens (100), automatisches und ohne Notwendigkeit eines Benutzereingriffs Auswählen eines oder mehrerer Teile des Volumens als Reaktion auf die dreidimensionale Live-Bildgebung des Volumens; und
automatisches und ohne Notwendigkeit eines Benutzereingriffs Durchführen einer Live-Bildgebung des einen oder der mehreren ausgewählten Abschnitte als Reaktion auf die Auswahl und mit einer Strahldichte, die höher als die räumliche Dichte ist;
wobei die Vorrichtung ferner konfiguriert ist, zum automatischen und ohne die Notwendigkeit eines Benutzereingriffs selektiven Unterbrechen (308) der Live-Bildgebung des einen oder der mehreren ausgewählten Abschnitte, um die dreidimensionale Live-Bildgebung des Volumens (S508) erneut auszuführen und, basierend auf der erneut ausgeführten dreidimensionalen Live-Bildgebung des Volumens, die Erkennung, das Auswählen und die Live-Bildgebung des einen oder der mehreren ausgewählten Abschnitten.

2. Vorrichtung nach Anspruch 1, wobei eine oder mehrere Abbildungsebenen (108, 160) jeweils den einen oder die mehreren Abschnitte umfassen.

3. Vorrichtung nach Anspruch 1, die so konfiguriert ist, dass das Unterbrechen periodisch (S540) erfolgt.

4. Vorrichtung nach Anspruch 1, konfiguriert zum Erfassen einer relativen Bewegung in Bezug auf jeweilige Positionen einer Bildgebungssonde und Körpergewebes (S538), wobei das Unterbrechen basierend auf der erfassten Bewegung ausgelöst wird.

5. Vorrichtung nach Anspruch 1, wobei die Volumenbildgebung Ultraschallbildgebung (120) und/oder Herzbildgebung (104) umfasst.

6. Vorrichtung nach Anspruch 1, wobei das Auswählen auf einem Optimalsicht-Kriterium basiert.

7. Vorrichtung nach Anspruch 6, wobei das Kriterium auf der Durchführung einer gezielten anatomischen Messung aus einem Bild basiert, das durch die Live-Bildgebung des einen oder der mehreren ausgewählten Abschnitte (S526) zu erzeugen ist.

8. Vorrichtung nach Anspruch 6, wobei das Auswählen gemäß dem Kriterium eine optimale Orientierung wählt.

9. Vorrichtung nach Anspruch 1, die ferner konfiguriert ist, zum automatischen und ohne Notwendigkeit eines Benutzereingriffs Berechnen eines Dopplerwinkels (152), basierend auf dem Anwenden eines anatomischen Modells auf mindestens eines von der Live-Bildgebung des einen oder der mehreren ausgewählten Abschnitte und der dreidimensionale Live-Bildgebung des Volumens.

10. Vorrichtung nach Anspruch 1, wobei das Auswählen derart ist, dass eine Abstandsmessung (424) zwischen vordefinierten anatomischen Punkten innerhalb des Körpergewebes optimiert wird, das durch Daten repräsentiert wird, die in der dreidimensionalen Live-Bildgebung des Volumens erfasst werden.

11. Vorrichtung nach Anspruch 1, die konfiguriert ist, um basierend auf der Anatomieerkennung, eine Messungs-Initialisierungsmarkierung (416, 420, 432, 436) abzuleiten und die abgeleitete Markierung anzuzeigen, um eine bildbasierte Messung innerhalb eines Bildes zu initialisieren, das durch die Live-Bildgebung des einen oder der mehreren ausgewählten Abschnitte erzeugt wird.

12. Vorrichtung nach Anspruch 11, wobei das Ableiten das Anwenden eines anatomischen Modells auf mindestens einen des einen oder der mehreren ausgewählten Abschnitte (204, 208) umfasst.

13. Vorrichtung nach Anspruch 1, die für das Auswählen so konfiguriert ist, dass ein Abschnitt aus dem einen oder den mehreren Abschnitten einen vordefinierten anatomischen Orientierungspunkt (424) innerhalb des Körpergewebes (408) überspannt, der durch Daten repräsentiert wird, die in der dreidimensionalen Live-Bildgebung des Volumens erfasst werden.

14. Vorrichtung nach Anspruch 1, wobei das Auswählen eine Betrachtungsebene senkrecht zu einer Mittellinie einer Aorta und entsprechend einem maximalen Durchmesser wählt, wenn die Betrachtungsebene entlang der Mittellinie verschoben wird.

15. Computerlesbares Medium (136) für eine Vorrichtung zur dreidimensionalen Live-Bildgebung eines Volumens mit sequentieller Erfassung mehrerer Strahlen, so dass die dreidimensionale Live-Bildgebung des Volumens mit einer räumlichen Dichte (156) der Strahlen durchgeführt wird, wobei das Medium Anweisungen umfasst, die von einem Prozessor ausführbar sind, um eine Vielzahl von Handlungen auszuführen, wobei unter der Vielzahl die Handlungen sind:
basierend auf einem Ergebnis der Anatomieerkennung aus der dreidimensionalen Live-Bildgebung eines Volumens, automatisches und ohne Notwendigkeit eines Benutzereingriffs Auswählen eines oder mehrerer Teile des Volumens als Reaktion auf die dreidimensionale Live-Bildgebung des Volumens; und
automatisches und ohne Notwendigkeit eines Benutzereingriffs Durchführen einer Live-Bildgebung des einen oder der mehreren ausgewählten Abschnitte als Reaktion auf die Auswahl und mit einer Strahldichte, die höher als die räumliche Dichte ist;
wobei unter der Vielzahl von Handlungen der weitere Vorgang des automatischen und ohne Notwendigkeit eines Benutzereingriffs selektiven Unterbrechens (308) der Live-Bildgebung des einen oder der mehreren ausgewählten Abschnitte ist, um die dreidimensionalen Live-Bildgebung des Volumens (S508) erneut auszuführen und, basierend auf der erneut ausgeführten dreidimensionalen Live-Bildgebung des Volumens, die Erkennung, das Auswählen und die Live-Bildgebung des einen oder der mehreren ausgewählten Abschnitte.

## Revendications

1. Dispositif de traitement d'image (146) configuré pour:
une imagerie tridimensionnelle en temps réel d'un volume avec acquisition séquentielle de faisceaux multiples de telle sorte que ladite imagerie tridimensionnelle en temps réel du volume de la matrice soit réalisée avec une densité spatiale (156) desdits faisceaux;
sur la base d'une reconnaissance anatomique (S510) à partir de ladite imagerie tridimensionnelle en temps réel du volume (100), automatiquement et sans intervention de l'utilisateur, sélectionner, en réponse à ladite imagerie tridimensionnelle en temps réel du volume, une ou plusieurs parties dudit volume; et
automatiquement et sans intervention de l'utilisateur, effectuer, en réponse à la sélection et avec une densité de faisceaux supérieure à ladite densité spatiale, une imagerie en temps réel de l'une ou plusieurs parties sélectionnées;
où ledit dispositif est en outre configuré pour automatiquement et sans intervention de l'utilisateur, interrompre sélectivement (308) l'imagerie en temps réel de l'une ou plusieurs parties sélectionnées pour réexécuter l'imagerie tridimensionnelle en temps réel du volume (S508) et, sur la base de l'imagerie tridimensionnelle en temps réel réexécutée du volume, ladite reconnaissance, ladite sélection et ladite imagerie en temps réel de l'une ou plusieurs parties sélectionnées.

2. Dispositif selon la revendication 1, un ou plusieurs plans d'imagerie (108, 160) comprenant respectivement lesdites une ou plusieurs parties.

3. Dispositif selon la revendication 1, configuré de telle sorte que ladite interruption se produise périodiquement (S540).

4. Dispositif selon la revendication 1, configuré pour détecter un mouvement relatif par rapport aux positions respectives d'une sonde d'imagerie et d'un tissu corporel (S538), ladite interruption étant déclenchée sur la base du mouvement détecté.

5. Dispositif selon la revendication 1, l'imagerie du volume comprenant une imagerie ultrasonore (120) et/ou une imagerie cardiaque (104).

6. Dispositif selon la revendication 1, ladite sélection étant basée sur un critère de vision optimale.

7. Dispositif selon la revendication 6, ledit critère étant basé sur la réalisation d'une mesure anatomique ciblée à partir d'une image à produire par l'imagerie en temps réel de l'une ou plusieurs parties sélectionnées (S526).

8. Dispositif selon la revendication 6, ladite sélection choisissant, en fonction dudit critère, une orientation optimale.

9. Dispositif selon la revendication 1, configuré en outre pour, automatiquement et sans intervention de l'utilisateur, calculer un angle Doppler (152) sur la base de l'application d'un modèle anatomique à au moins l'une entre l'imagerie en temps réel de l'une ou plusieurs parties sélectionnées et l'imagerie tridimensionnelle en temps réel du volume.

10. Dispositif selon la revendication 1, ladite sélection étant telle qu'elle optimise une mesure de distance (424), entre des points anatomiques prédéfinis, à l'intérieur du tissu corporel représenté par des données acquises dans l'imagerie tridimensionnelle en temps réel du volume.

11. Dispositif selon la revendication 1, configuré pour, sur la base de la reconnaissance de l'anatomie, dériver un indice d'initialisation de mesure (416, 420, 432, 436), et pour afficher l'indice dérivé pour initialiser une mesure basée sur une image dans une image produite par l'imagerie en temps réel de l'une ou plusieurs parties sélectionnées.

12. Dispositif selon la revendication 11, ladite dérivation comprenant l'application d'un modèle anatomique sur au moins l'une desdites une ou plusieurs parties sélectionnées (204, 208).

13. Dispositif selon la revendication 1, configuré pour ladite sélection de telle sorte qu'une partie parmi lesdites une ou plusieurs parties couvre un repère anatomique prédéfini (424) à l'intérieur du tissu corporel (408) qui est représenté par des données acquises dans l'imagerie tridimensionnelle en temps réel du volume.

14. Dispositif selon la revendication 1, ladite sélection choisissant un plan d'observation perpendiculaire à une ligne médiane d'une aorte et correspondant à un diamètre maximum, lors du déplacement du plan d'observation le long de cette ligne médiane.

15. Support lisible par ordinateur (136) pour un dispositif d'imagerie tridimensionnelle en temps réel d'un volume avec acquisition séquentielle de plusieurs faisceaux de telle sorte que ladite imagerie tridimensionnelle en temps réel du volume est réalisée avec une densité spatiale (156) desdits faisceaux, ledit support comprenant des instructions exécutables par un processeur pour effectuer une pluralité d'actes, parmi ladite pluralité il y a les actes de:
sur la base d'un résultat de reconnaissance anatomique à partir de ladite imagerie tridimensionnelle en temps réel d'un volume, sélectionner, automatiquement et sans intervention de l'utilisateur, en réponse à ladite imagerie tridimensionnelle en temps réel du volume, une ou plusieurs parties dudit volume; et
automatiquement et sans intervention de l'utilisateur, effectuer, en réponse à la sélection et avec une densité de faisceaux supérieure à ladite densité spatiale, une imagerie en temps réel de l'une ou plusieurs parties sélectionnées;
où, parmi ladite pluralité d'actes, il y a l'acte supplémentaire d'interrompre automatiquement et sans intervention de l'utilisateur (308) de manière sélective l'imagerie en temps réel de l'une ou des plusieurs parties sélectionnées pour réexécuter l'imagerie tridimensionnelle en temps réel du volume (S508) et, sur la base de l'imagerie tridimensionnelle en temps réel réexécutée du volume, ladite reconnaissance, ladite sélection et ladite imagerie en temps réel de l'une ou plusieurs parties sélectionnées.
